# EUROPEAN PATENT APPLICATION

(11) **EP 3 871 600 A1**
(43) Date of publication of application: **01.09.2021**
(21) Application number: 21159739.8
(22) Date of filing: 26.02.2021
(51) Int. Cl.: A61B 5/087, A61M 16/06, G16H 20/10

(54) **SYSTEMS AND METHODS FOR MONITORING AND MANAGEMENT OF A PATIENT'S OXYGEN THERAPY AND BREATHING**

(30) Priority: 26.02.2020 US 202016801455
(71) Applicant: Soifer, Gregg, Dothan, Alabama 36305 (US)
(72) Inventor: Soifer, Gregg, Dothan, Alabama 36305 (US)
(74) Representative: Loo, Chi Ching

(57) **Abstract**

Disclosed herein is a method for monitoring and management of a patient's oxygen therapy and breathing. Accordingly, the method may include a step of receiving, using a communication device (206), at least one sensor data from a sensor device (202). Further, the method may include a step of analyzing, using a processing device (204), the at least one sensor data. Further, the method may include a step of generating, using the processing device (204), a notification based on the analyzing. Further, the method may include a step of transmitting, using the communication device (206), the notification to at least one external device . Further, the method may include a step of storing, using a storage device (208), the notification.

## Description

### FIELD OF THE INVENTION

Generally, the present disclosure relates to the field of data processing. More specifically, the present disclosure relates to systems and methods for monitoring and management of a patient's oxygen therapy and breathing, in real time and for retrospective review.

### BACKGROUND OF THE INVENTION

Oxygen therapy is prescribed for people who can't get enough oxygen on their own. This is often because of lung conditions that prevent the lungs from absorbing oxygen including chronic obstructive pulmonary disease (COPD), asthma, chronic bronchitis amongst others. Doctors give a prescription that spells out how much oxygen is needed per minute and when the need to get it. Some people may need oxygen therapy only when they exercise or sleep, while others may need it all day long. However, there are challenges and problems in managing the oxygen therapy within health facilities and much more when administered at home. These challenges result in a patient not having the required amount of oxygen intake, or having too much that results in oxygen intoxication, affecting the central nervous system, lungs, and eyes leading to cell damage and death unbeknownst to the healthcare providers, or the patients themselves.

Therefore, there is a need for improved systems and methods for the monitoring and management of a patient's oxygen therapy and breathing, that may overcome one or more of the above-mentioned problems and/or limitations.

### SUMMARY OF THE INVENTION

This summary is provided to introduce a selection of concepts in a simplified form, that are further described below in the Detailed Description. This summary is not intended to identify key features or essential features of the claimed subject matter. Nor is this summary intended to be used to limit the claimed subject matter's scope.

Disclosed herein is a system for monitoring and management of a patient's oxygen therapy and breathing, in accordance with some embodiments. Accordingly, the system may include a sensor device, a processing device, a communication device, and a storage device. Further, the sensor device may be configured for detecting a flow. Further, the sensor device may be configured to be fluidly couplable with a nasal cannula. Further, the nasal cannula may be configured for dispensing oxygen to a nasal cavity of a patient. Further, the sensor device may be configured for generating at least one sensor data based on detection of the flow. Further, the processing device may be communicatively coupled with the sensor device. Further, the processing device may be configured for analyzing the at least one sensor data. Further, the processing device may be configured for generating a notification based on the analyzing. Further, the communication device may be communicatively coupled with the processing device. Further, the communication device may be configured for transmitting the notification to at least one external device. Further, the storage device may be communicatively coupled with the processing device. Further, the storage device may be configured for storing the notification.

Further disclosed herein is a method for monitoring and management of a patient's oxygen therapy and breathing, in accordance with some embodiments. Accordingly, the method may include a step of receiving, using a communication device, at least one sensor data from a sensor device. Further, the method may include a step of analyzing, using a processing device, the at least one sensor data. Further, the method may include a step of generating, using the processing device, a notification based on the analyzing. Further, the method may include a step of transmitting, using the communication device, the notification to at least one external device. Further, the method may include a step of storing, using a storage device, the notification.

Both the foregoing summary and the following detailed description provide examples and are explanatory only. Accordingly, the foregoing summary and the following detailed description should not be considered to be restrictive. Further, features or variations may be provided in addition to those set forth herein. For example, embodiments may be directed to various feature combinations and sub-combinations described in the detailed description.

### BRIEF DESCRIPTION OF DRAWINGS

The accompanying drawings, which are incorporated in and constitute a part of this disclosure, illustrate various embodiments of the present disclosure. The drawings contain representations of various trademarks and copyrights owned by the Applicants. In addition, the drawings may contain other marks owned by third parties and are being used for illustrative purposes only. All rights to various trademarks and copyrights represented herein, except those belonging to their respective owners, are vested in and the property of the applicants. The applicants retain and reserve all rights in their trademarks and copyrights included herein, and grant permission to reproduce the material only in connection with reproduction of the granted patent and for no other purpose.

Furthermore, the drawings may contain text or captions that may explain certain embodiments of the present disclosure. This text is included for illustrative, non-limiting, explanatory purposes of certain embodiments detailed in the present disclosure.
FIG. **1** is an illustration of an online platform consistent with various embodiments of the present disclosure.
FIG. **2** is a block diagram of a system for monitoring and management of a patient's oxygen therapy and breathing, in accordance with some embodiments.
FIG. **3** is a block diagram of a system for monitoring and management of a patient's oxygen therapy and breathing, in accordance with some embodiments.
FIG. **4** is a flowchart of a method for monitoring and management of a patient's oxygen therapy and breathing, in accordance with some embodiments.
FIG. **5** is a flowchart of a method for facilitating the generation of an alert, in accordance with some embodiments.
FIG. **6** is a flowchart of a method for facilitating the generation of a second notification, in accordance with some embodiments.
FIG. **7** is a flowchart of a method for facilitating the generation of a therapy program, in accordance with some embodiments.
FIG. **8** is a flowchart of a method for facilitating the generation of pressure data, in accordance with some embodiments.
FIG. **9** is a top perspective view of a nasal cannula, in accordance with some embodiments.
FIG. **10** is a front view of a user with a nasal cannula in nostrils and tubing secured around ears, in accordance with some embodiments.
FIG. **11** is a side view of a user with a nasal cannula in nostrils and tubing secured around ears, in accordance with some embodiments.
FIG. **12** is a top view of an oxygen delivery monitor (ODM), in accordance with some embodiments.
FIG. **13** is a front view of the ODM, in accordance with some embodiments.
FIG. **14** is a rear view of the ODM, in accordance with some embodiments.
FIG. **15** is a block diagram of a system for monitoring and management of a patient's oxygen therapy and breathing, in real time and for retrospective review, in accordance with some embodiments.
FIG. **16** is a block diagram of a system to facilitate monitoring and management of a patient's oxygen therapy and breathing, in real time and for retrospective review by an oxygen flow device, in accordance with some embodiments.
FIG. **17** is a block diagram of a system to facilitate data transmission associated with monitoring and management of a patient's oxygen therapy and breathing, in real time and for retrospective review, in accordance with some embodiments.
FIG. **18** is a block diagram of a system including a central device interacting with an oxygen flow device and other devices to facilitate monitoring and management of a patient's oxygen therapy and breathing, in real time and for retrospective review, in accordance with some embodiments.
FIG. **19** is a block diagram of a system including other devices interacting with a central device to facilitate monitoring and management of a patient's oxygen therapy and breathing, in real time and for retrospective review, in accordance with some embodiments.
FIG. **20** is a block diagram of an oxygen flow device to facilitate monitoring and management of a patient's oxygen therapy and breathing, in real time and for retrospective review, in accordance with some embodiments.
FIG. **21** is a block diagram of a system including a central device interacting with an oxygen flow device and other devices to facilitate monitoring and management of a patient's oxygen therapy and breathing, in real time and for retrospective review, in accordance with some embodiments.
FIG. **22** is a block diagram of a system to facilitate monitoring and management of a patient's oxygen therapy and breathing, in real time and for retrospective review using other devices, in accordance with some embodiments.
FIG. **23** is a block diagram of a computing device for implementing the methods disclosed herein, in accordance with some embodiments.

### DETAILED DESCRIPTION OF THE INVENTION

As a preliminary matter, it will readily be understood by one having ordinary skill in the relevant art that the present disclosure has broad utility and application. As should be understood, any embodiment may incorporate only one or a plurality of the above-disclosed aspects of the disclosure and may further incorporate only one or a plurality of the above-disclosed features. Furthermore, any embodiment discussed and identified as being "preferred" is considered to be part of a best mode contemplated for carrying out the embodiments of the present disclosure. Other embodiments also may be discussed for additional illustrative purposes in providing a full and enabling disclosure. Moreover, many embodiments, such as adaptations, variations, modifications, and equivalent arrangements, will be implicitly disclosed by the embodiments described herein and fall within the scope of the present disclosure.

Accordingly, while embodiments are described herein in detail in relation to one or more embodiments, it is to be understood that this disclosure is illustrative and exemplary of the present disclosure, and are made merely for the purposes of providing a full and enabling disclosure. The detailed disclosure herein of one or more embodiments is not intended, nor is to be construed, to limit the scope of patent protection afforded in any claim of a patent issuing here from, which scope is to be defined by the claims and the equivalents thereof. It is not intended that the scope of patent protection be defined by reading into any claim limitation found herein and/or issuing here from that does not explicitly appear in the claim itself.

Thus, for example, any sequence(s) and/or temporal order of steps of various processes or methods that are described herein are illustrative and not restrictive. Accordingly, it should be understood that, although steps of various processes or methods may be shown and described as being in a sequence or temporal order, the steps of any such processes or methods are not limited to being carried out in any particular sequence or order, absent an indication otherwise. Indeed, the steps in such processes or methods generally may be carried out in various different sequences and orders while still falling within the scope of the present disclosure. Accordingly, it is intended that the scope of patent protection is to be defined by the issued claim(s) rather than the description set forth herein.

Additionally, it is important to note that each term used herein refers to that which an ordinary artisan would understand such term to mean based on the contextual use of such term herein. To the extent that the meaning of a term used herein-as understood by the ordinary artisan based on the contextual use of such term-differs in any way from any particular dictionary definition of such term, it is intended that the meaning of the term as understood by the ordinary artisan should prevail.

Furthermore, it is important to note that, as used herein, "a" and "an" each generally denotes "at least one," but does not exclude a plurality unless the contextual use dictates otherwise. When used herein to join a list of items, "or" denotes "at least one of the items," but does not exclude a plurality of items of the list. Finally, when used herein to join a list of items, "and" denotes "all of the items of the list."

The following detailed description refers to the accompanying drawings. Wherever possible, the same reference numbers are used in the drawings and the following description to refer to the same or similar elements. While many embodiments of the disclosure may be described, modifications, adaptations, and other implementations are possible. For example, substitutions, additions, or modifications may be made to the elements illustrated in the drawings, and the methods described herein may be modified by substituting, reordering, or adding stages to the disclosed methods. Accordingly, the following detailed description does not limit the disclosure. Instead, the proper scope of the disclosure is defined by the claims found herein and/or issuing here from. The present disclosure contains headers. It should be understood that these headers are used as references and are not to be construed as limiting upon the subjected matter disclosed under the header.

The present disclosure includes many aspects and features. Moreover, while many aspects and features relate to, and are described in the context of systems and methods for monitoring and management of a patient's oxygen therapy and breathing, embodiments of the present disclosure are not limited to use only in this context.

In general, the method disclosed herein may be performed by one or more computing devices. For example, in some embodiments, the method may be performed by a server computer in communication with one or more client devices over a communication network such as, for example, the Internet. In some other embodiments, the method may be performed by one or more of at least one server computer, at least one client device, at least one network device, at least one sensor and at least one actuator. Examples of the one or more client devices and/or the server computer may include, a desktop computer, a laptop computer, a tablet computer, a personal digital assistant, a portable electronic device, a wearable computer, a smart phone, an Internet of Things (IoT) device, a smart electrical appliance, a video game console, a rack server, a super-computer, a mainframe computer, mini-computer, micro-computer, a storage server, an application server (e.g. a mail server, a web server, a real-time communication server, an FTP server, a virtual server, a proxy server, a DNS server, etc.), a quantum computer, and so on. Further, one or more client devices and/or the server computer may be configured for executing a software application such as, for example, but not limited to, an operating system (e.g. Windows, Mac OS, Unix, Linux, Android, etc.) in order to provide a user interface (e.g. GUI, touch-screen based interface, voice-based interface, gesture-based interface etc.) for use by the one or more users and/or a network interface for communicating with other devices over a communication network. Accordingly, the server computer may include a processing device configured for performing data processing tasks such as, for example, but not limited to, analyzing, identifying, determining, generating, transforming, calculating, computing, compressing, decompressing, encrypting, decrypting, scrambling, splitting, merging, interpolating, extrapolating, redacting, anonymizing, encoding and decoding. Further, the server computer may include a communication device configured for communicating with one or more external devices. The one or more external devices may include, for example, but are not limited to, a client device, a third-party database, public database, a private database and so on. Further, the communication device may be configured for communicating with the one or more external devices over one or more communication channels. Further, the one or more communication channels may include a wireless communication channel and/or a wired communication channel. Accordingly, the communication device may be configured for performing one or more of transmitting and receiving of information in electronic form. Further, the server computer may include a storage device configured for performing data storage and/or data retrieval operations. In general, the storage device may be configured for providing reliable storage of digital information. Accordingly, in some embodiments, the storage device may be based on technologies such as, but not limited to, data compression, data backup, data redundancy, deduplication, error correction, data finger-printing, role-based access control, and so on.

Further, one or more steps of the method disclosed herein may be initiated, maintained, controlled and/or terminated based on a control input received from one or more devices operated by one or more users such as, for example, but not limited to, an end-user, an admin, a service provider, a service consumer, an agent, a broker and a representative thereof. Further, the user as defined herein may refer to a human, an animal or an artificially intelligent being in any state of existence, unless stated otherwise, elsewhere in the present disclosure. Further, in some embodiments, the one or more users may be required to successfully perform authentication in order for the control input to be effective. In general, a user of the one or more users may perform authentication based on the possession of a secret human-readable secret data (e.g. username, password, passphrase, PIN, secret question, secret answer etc.) and/or possession of a machine-readable secret data (e.g. encryption key, decryption key, bar codes, etc.) and/or possession of one or more embodied characteristics unique to the user (e.g. biometric variables such as, but not limited to, fingerprint, palm-print, voice characteristics, behavioral characteristics, facial features, iris pattern, heart rate variability, evoked potentials, brain waves, and so on) and/or possession of a unique device (e.g. a device with a unique physical and/or chemical and/or biological characteristic, a hardware device with a unique serial number, a network device with a unique IP/MAC address, a telephone with a unique phone number, a smartcard with an authentication token stored thereupon, etc.). Accordingly, the one or more steps of the method may include communicating (e.g. transmitting and/or receiving) with one or more sensor devices and/or one or more actuators in order to perform authentication. For example, the one or more steps may include receiving, using the communication device, the secret human-readable data from an input device such as, for example, a keyboard, a keypad, a touch-screen, a microphone, a camera and so on. Likewise, the one or more steps may include receiving, using the communication device, the one or more embodied characteristics from one or more biometric sensors.

Further, one or more steps of the method may be automatically initiated, maintained and/or terminated based on one or more predefined conditions. In an instance, the one or more predefined conditions may be based on one or more contextual variables. In general, the one or more contextual variables may represent a condition relevant to the performance of the one or more steps of the method. The one or more contextual variables may include, for example, but are not limited to, location, time, identity of a user associated with a device (e.g. the server computer, a client device, etc.) corresponding to the performance of the one or more steps, environmental variables (e.g. temperature, humidity, pressure, wind speed, lighting, sound, etc.) associated with a device corresponding to the performance of the one or more steps, physical state and/or physiological state and/or psychological state of the user, physical state (e.g. motion, direction of motion, orientation, speed, velocity, acceleration, trajectory, etc.) of the device corresponding to the performance of the one or more steps and/or semantic content of data associated with the one or more users. Accordingly, the one or more steps may include communicating with one or more sensors and/or one or more actuators associated with the one or more contextual variables. For example, the one or more sensors may include, but are not limited to, a timing device (e.g. a real-time clock), a location sensor (e.g. a GPS receiver, a GLONASS receiver, an indoor location sensor etc.), a biometric sensor (e.g. a fingerprint sensor), an environmental variable sensor (e.g. temperature sensor, humidity sensor, pressure sensor, etc.) and a device state sensor (e.g. a power sensor, a voltage/current sensor, a switch-state sensor, a usage sensor, etc. associated with the device corresponding to performance of the or more steps).

Further, the one or more steps of the method may be performed one or more number of times. Additionally, the one or more steps may be performed in any order other than as exemplarily disclosed herein, unless explicitly stated otherwise, elsewhere in the present disclosure. Further, two or more steps of the one or more steps may, in some embodiments, be simultaneously performed, at least in part. Further, in some embodiments, there may be one or more time gaps between performance of any two steps of the one or more steps.

Further, in some embodiments, the one or more predefined conditions may be specified by the one or more users. Accordingly, the one or more steps may include receiving, using the communication device, the one or more predefined conditions from one or more and devices operated by the one or more users. Further, the one or more predefined conditions may be stored in the storage device. Alternatively, and/or additionally, in some embodiments, the one or more predefined conditions may be automatically determined, using the processing device, based on historical data corresponding to performance of the one or more steps. For example, the historical data may be collected, using the storage device, from a plurality of instances of performance of the method. Such historical data may include performance actions (e.g. initiating, maintaining, interrupting, terminating, etc.) of the one or more steps and/or the one or more contextual variables associated therewith. Further, machine learning may be performed on the historical data in order to determine the one or more predefined conditions. For instance, machine learning on the historical data may determine a correlation between one or more contextual variables and performance of the one or more steps of the method. Accordingly, the one or more predefined conditions may be generated, using the processing device, based on the correlation.

Further, one or more steps of the method may be performed at one or more spatial locations. For instance, the method may be performed by a plurality of devices interconnected through a communication network. Accordingly, in an example, one or more steps of the method may be performed by a server computer. Similarly, one or more steps of the method may be performed by a client computer. Likewise, one or more steps of the method may be performed by an intermediate entity such as, for example, a proxy server. For instance, one or more steps of the method may be performed in a distributed fashion across the plurality of devices in order to meet one or more objectives. For example, one objective may be to provide load balancing between two or more devices. Another objective may be to restrict a location of one or more of an input data, an output data and any intermediate data therebetween corresponding to one or more steps of the method. For example, in a client-server environment, sensitive data corresponding to a user may not be allowed to be transmitted to the server computer. Accordingly, one or more steps of the method operating on the sensitive data and/or a derivative thereof may be performed at the client device.

### Overview:

The present disclosure describes systems and methods for monitoring and management of a patient's oxygen therapy and breathing. Further, the present disclosure may include an oxygen delivery monitor (ODM) that monitors the delivery of supplemental oxygen through a nasal cannula to hospital, home-care, nursing home, and other patient settings. Further, the ODM determines if the nasal cannula is delivering the prescribed oxygen flow into a patient's nasal cavity or if the cannula has become dislodged or, if not dislodged, if the patient is experiencing abnormal breathing. In either case, the ODM transmits notification via Bluetooth signal coupled with a smartphone and/or computer apps to alert the on-duty nurse, nurses' station or caregiver of a problem so that remedial action may be taken.

Further, the present disclosure relates to monitoring the delivery of supplemental oxygen via a nasal cannula to patients in a hospital, nursing home, home-care, or other setting. Further, the present disclosure may detect if the nasal cannula has become dislodged or if the patient is experiencing abnormal breathing. Further, the ODM may alert the on-duty nurse or nurses' station in the hospital setting or the caregiver in the home-care setting or other setting, if such an event occurs so that remedial action may be taken. Further, the present disclosure may also provide a record of the event.

Further, a nasal cannula may be commonly used to deliver supplemental oxygen to patients in the hospital, nursing home, home-care, or other settings who are in need of respiratory assistance. A common problem experienced with the nasal cannula is that one or both prongs become dislodged from the patient's nostrils, depriving the patient of his/her prescribed respiratory assistance. The dislodged cannula continues to deliver oxygen but not to the benefit of the patient, and the event goes undetected and unrecorded until the dislodged cannula is sighted and corrected by a nurse or caregiver.

Some of the obvious and non-obvious oxygen therapy challenges are as follows:
- informing helper/patient to start/end the therapy session based on the doctor's prescribed date/time and duration of the therapy session/s.
- gathering of data during the whole span of the Oxygen Therapy (OT) session, such as:
   - oxygen flow rate/settings (consistency and occurrence of fluctuations)
   - length of actual/performed therapy
   - the patient's breathing pattern during the session (normal and abnormal)
- comparing/analyzing the data of the actual OT session vs. the prescribed therapy
- saving and archiving of current/old oxygen therapy data
- sending real-time alerts and notification messages when an incident/problem happens, such as:
   - when the nasal cannula is detached/displaced from the nose
   - when the oxygen regulator is not giving the prescribed amount of oxygen
   - when critical breathing patterns are detected, like:
   - fast/shallow breathing
   - stop breathing (like in sleep apnea)
   - when the start/end time of the OT session is not met

Although the above can be done manually with due diligence and with dedicated help from an abled person, the ODM may do the above with a higher level of accuracy and consistency.

Further, the present disclosure may be heavily technology-based (using the latest available hardware and software). Further, the present disclosure may look similar to existing oxygen flow inventions due to hardware compositions (sensors, peripheral, etc.), network connectivity, and external devices. Further, the present disclosure differs from others is the way the hardware components are assembled, the functionality each hardware executes, and the timing/sequence of events that triggers the hardware.

Further, the present disclosure uncovered grey areas where other oxygen flow devices fail, particularly in the delivery of 360-view of Oxygen Therapy Sessions. Thus, the present disclosure conceptualized an Oxygen Therapy Process/Management that may be unique as of this writing:
- automates the process of monitoring, recording, analyzing, and reporting of Oxygen Therapy sessions;
- allows doctors/physicians to create and integrate Oxygen Therapy Program for individual patients within an automated system.
- learns the patient's normal breathing pattern even if the pattern is unusual (abnormal) due to existing health condition, and use that learned pattern as the base for the therapy session.
- differentiates normal and abnormal situations during therapy, and raise corresponding alert levels in real-time.
- detects non-compliance of therapy schedule, and raise corresponding notification levels.
- allows multiple oxygen flow devices to connect to one central device (many-to-one topology), thereby a centralized view is possible for multi-tenant health facilities.
- saves data in the most desired granular details, thus enabling doctors/physicians to dig into particular details (or data segment) of the Oxygen Therapy session.
- a non-intrusive device that allows the normal flow of oxygen from source to the patient, even when the device itself became unfunctional, such as:
   - when the power source trips down thus, turning off the device unexpectedly
   - when the device is cut-off from the network/connection to the central device (off-line)
   - when the firmware/software of the device stops from its normal operation

There may be existing oxygen flow devices which appears to be similar to the present disclosure but there are details that must be highlighted:

| CAN DO | CAN'T DO |
|---|---|
| (Functionality) measure the flow of oxygen (passive) | control the flow of oxygen (active) |
| (Functionality) monitor the airflow and log the data from start to end of the oxygen therapy (non-intrusive) | use to conduct forced therapy (intrusive) |
| (Physical layout) connected between existing oxygen regulator and off-the-shelf nasal cannula | as a replacement for oxygen concentrator/regulator |
| | as a replacement for ordinary nasal cannula (custom with built-in sensors) |
| (Network) connects to nurse-station device (desktop/ laptop/mobile/IoT) or to the home's designated device (tablet/mobile) | operates without connectivity to external devices (stand-alone) |
| (Network) multiple oxygen flow devices can connect to 1 central external/remote device (many-to-1 connectivity) | |
| (Connection) use long-range wireless modules like Wi-Fi, Lora, LPWAN, NB-IoT, and the upcoming technologies like LIFI and WEIGHTLESS | |
| (Software Framework) use Artificial Intelligence (AI), computer vision, graph, plotters and other related software/ modules for signal and wave-form analysis. | |

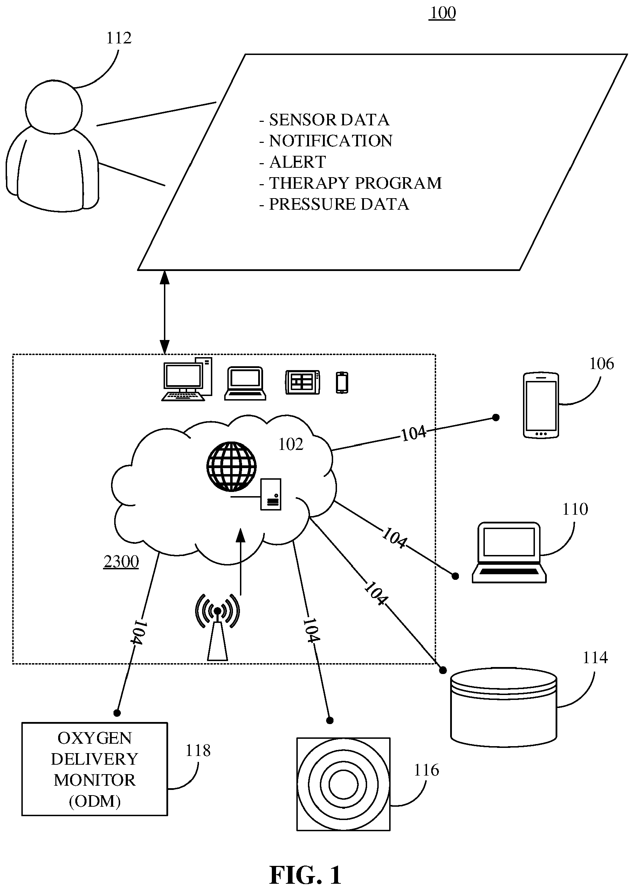

Referring now to figures, FIG. 1 is an illustration of an online platform 100 consistent with various embodiments of the present disclosure. By way of non-limiting example, the online platform 100 to facilitate administration of oxygen therapy may be hosted on a centralized server 102, such as, for example, a cloud computing service. The centralized server 102 may communicate with other network entities, such as, for example, a mobile device 106 (such as a smartphone, a laptop, a tablet computer, etc.), other electronic devices 110 (such as desktop computers, server computers, etc.), databases 114, sensors 116, and an oxygen delivery monitor (ODM) 118 over a communication network 104, such as, but not limited to, the Internet. Further, users of the online platform 100 may include relevant parties such as, but not limited to, end-users, administrators, service providers, service consumers and so on. Accordingly, in some instances, electronic devices operated by the one or more relevant parties may be in communication with the platform.

A user 112, such as the one or more relevant parties, may access online platform 100 through a web-based software application or browser. The web-based software application may be embodied as, for example, but not be limited to, a website, a web application, a desktop application, and a mobile application compatible with a computing device 2300.

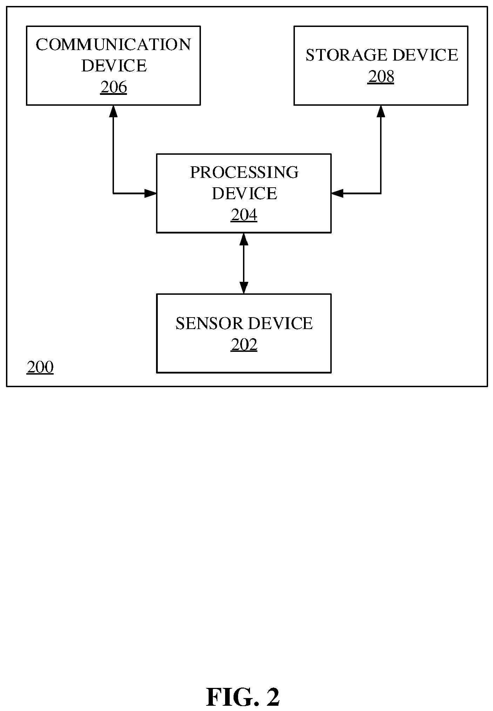

FIG. **2** is a block diagram of a system 200 for monitoring and management of a patient's oxygen therapy and breathing, in accordance with some embodiments. Accordingly, the system 200 may include a sensor device 202, a processing device 204, a communication device 206, and a storage device 208.

Further, the sensor device 202 may be configured for detecting a flow. Further, the sensor device 202 may be configured to be fluidly couplable with a nasal cannula. Further, the nasal cannula may be configured for dispensing oxygen to a nasal cavity of a patient. Further, the sensor device 202 may be configured for generating at least one sensor data based on detection of the flow.

Further, the processing device 204 may be communicatively coupled with the sensor device 202. Further, the processing device 204 may be configured for analyzing the at least one sensor data. Further, the processing device 204 may be configured for generating a notification based on the analyzing.

Further, the communication device 206 may be communicatively coupled with the processing device 204. Further, the communication device 206 may be configured for transmitting the notification to at least one external device.

Further, the storage device 208 may be communicatively coupled with the processing device 204. Further, the storage device 208 may be configured for storing the notification.

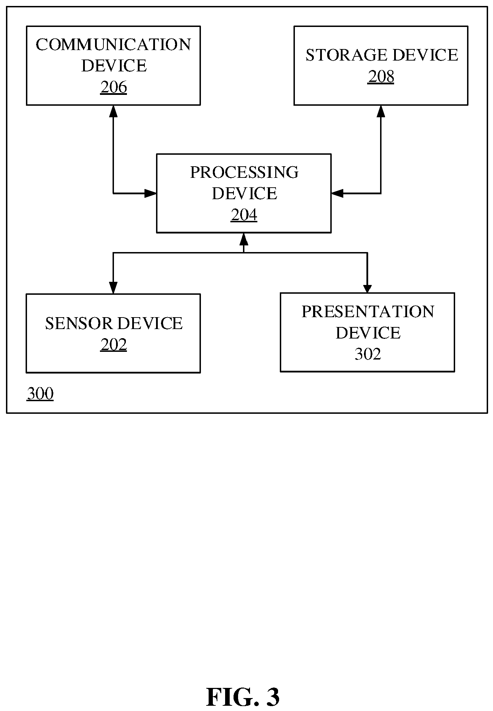

Further, in some embodiments, the system 200 further may include a presentation device 302 communicatively coupled with the processing device 204, as shown in FIG. 3. Further, the presentation device 302 may be configured for presenting the notification.

Further, in some embodiments, the processing device 204 may be configured for determining a patient respiration data based on the analyzing. Further, the processing device 204 may be configured for analyzing the patient respiration data. Further, the processing device 204 may be configured for generating an alert based on the analyzing of the patient respiration data. Further, the communication device 206 may be configured for transmitting the alert to the at least one external device.

Further, in some embodiments, the storage device 208 may be configured for retrieving a patient data. Further, the processing device 204 may be configured for processing at least one of the patient data and the patient respiration data. Further, the processing device 204 may be configured for generating a second notification based on the processing.

Further, in some embodiments, the processing device 204 may be configured for generating a therapy program based on the processing. Further, the communication device 206 may be configured for transmitting the therapy program to at least one of at least one patient device and the at least one external device.

Further, in some embodiments, the system 200 may be compliant with at least one Health Insurance Portability and Accountability Act (HIPPA) regulation. Further, the processing device 204 may be configured for deleting at least one of the at least one sensor data, the patient data, and the patient respiration data. Further, the deletion may be subsequent to the analyzing.

Further, in some embodiments, the analyzing may be further based on the therapy program. Further, the notification may include a compliance notification. Further, the communication device 206 may be configured for transmitting the compliance notification to at least one of the at least one patient device and the at least one external device.

Further, in some embodiments, the processing device 204 may be configured for generating a pressure data based on the analyzing. Further, the communication device 206 may be configured for transmitting the pressure data to the at least one external device.

Further, in some embodiments, the sensor device 202 may include a microprocessor, a wireless module, and a memory. Further, the microprocessor may be configured for performing at least one computational process. Further, the wireless module may be configured for connecting the sensor device 202 to the at least one external device. Further, the memory may be configured for storing a data associated with the sensor device 202.

Further, in some embodiments, the sensor device 202 may include a non-intrusive device. Further, the non-intrusive device may be configured for allowing the flow upon failure of the sensor device 202.

Further, the system 200 may facilitate monitoring and management of a patient's oxygen therapy and breathing, in real time and for retrospective review.

FIG. **3** is a block diagram of a system 300 for monitoring and management of a patient's oxygen therapy and breathing, in accordance with some embodiments.

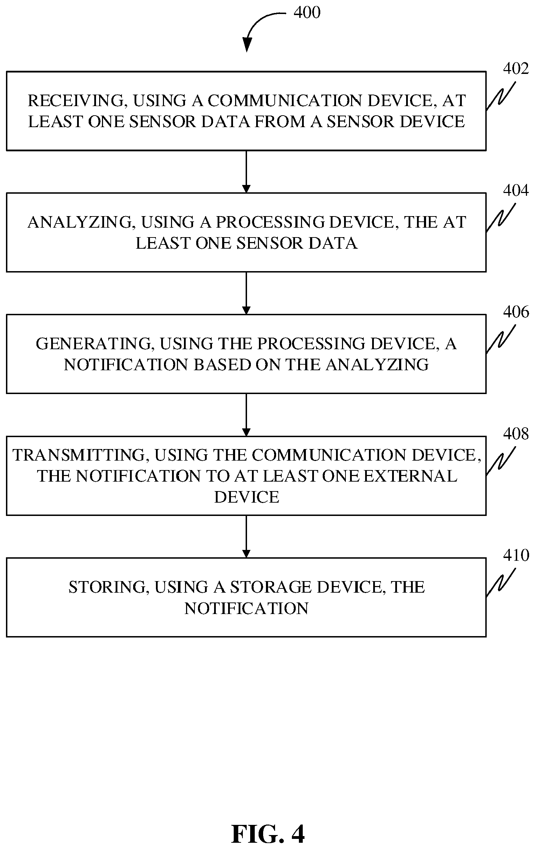

FIG. **4** is a flowchart of a method 400 for monitoring and management of a patient's oxygen therapy and breathing, in accordance with some embodiments. Accordingly, at 402, the method 400 may include a step of receiving, using a communication device, at least one sensor data from a sensor device. Further, the sensor device configured for detecting a flow. Further, the sensor device may be configured to be fluidly couplable with a nasal cannula. Further, the nasal cannula may be configured for dispensing oxygen to a nasal cavity of a patient.

Further, at 404, the method 400 may include a step of analyzing, using a processing device, the at least one sensor data.

Further, at 406, the method 400 may include a step of generating, using the processing device, a notification based on the analyzing.

Further, at 408, the method 400 may include a step of transmitting, using the communication device, the notification to at least one external device.

Further, at 410, the method 400 may include a step of storing, using a storage device, the notification.

Further, in some embodiments, the method 400 may include a step of transmitting, using the communication device, the notification to a presentation device. Further, the presentation device may be configured for presenting the notification.

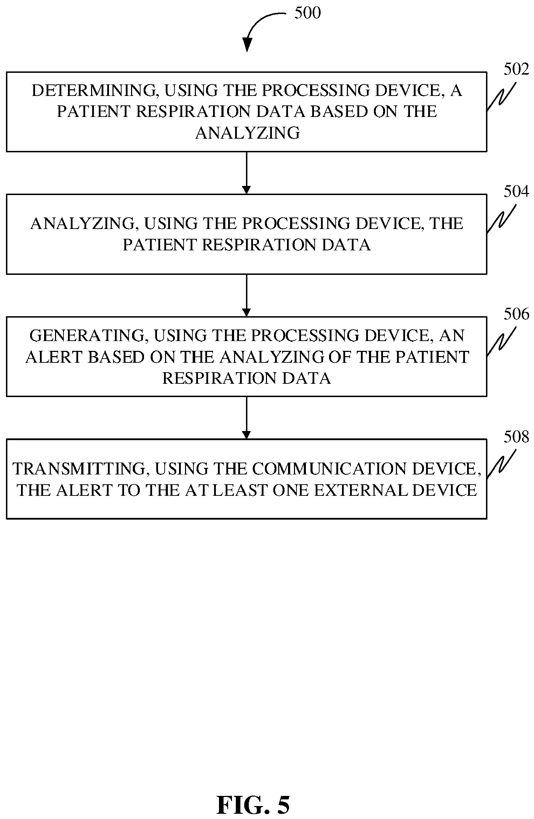

FIG. **5** is a flowchart of a method 500 for facilitating the generation of an alert, in accordance with some embodiments. Further, at 502, the method 500 may include a step of determining, using the processing device, a patient respiration data based on the analyzing.

Further, at 504, the method 500 may include a step of analyzing, using the processing device, the patient respiration data.

Further, at 506, the method 500 may include a step of generating, using the processing device, an alert based on the analyzing of the patient respiration data.

Further, at 508, the method 500 may include a step of transmitting, using the communication device, the alert to the at least one external device.

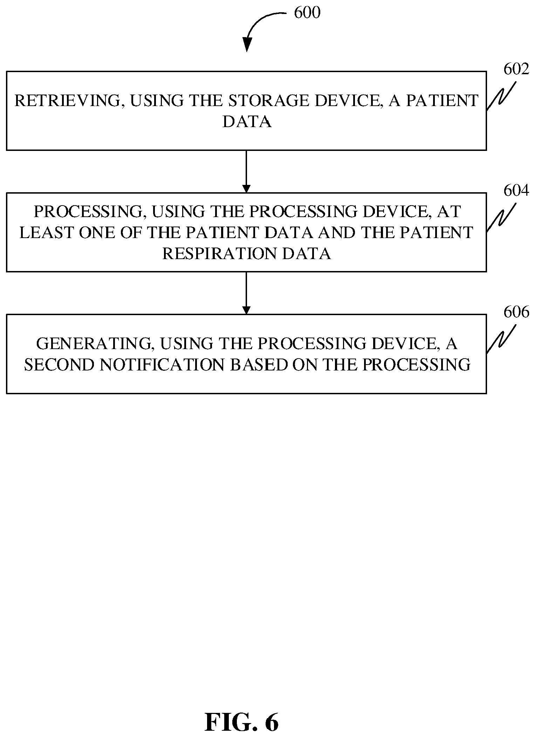

FIG. **6** is a flowchart of a method 600 for facilitating the generation of a second notification, in accordance with some embodiments. Further, at 602, the method 600 may include a step of retrieving, using the storage device, a patient data.

Further, at 604, the method 600 may include a step of processing, using the processing device, at least one of the patient data and the patient respiration data.

Further, at 606, the method 600 may include a step of generating, using the processing device, a second notification based on the processing.

Further, in some embodiments, the method 600 may be compliant with at least one Health Insurance Portability and Accountability Act (HIPPA) regulation. Further, the method 600 further may include deleting, using the processing device, at least one of the at least one sensor data, the patient data, and the patient respiration data. Further, the deleting is subsequent to the analyzing.

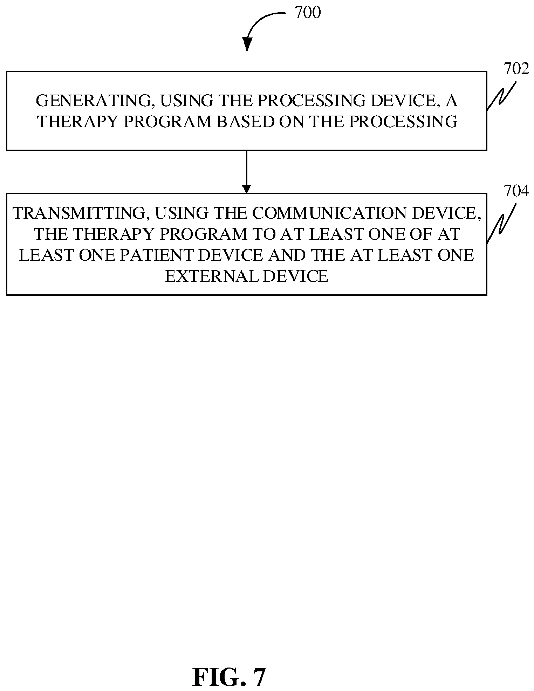

FIG. 7 is a flowchart of a method 700 for facilitating the generation of a therapy program, in accordance with some embodiments. Further, at 702, the method 700 may include a step of generating, using the processing device, a therapy program based on the processing.

Further, at 704, the method 700 may include a step of transmitting, using the communication device, the therapy program to at least one of at least one patient device and the at least one external device.

Further, in some embodiments, the analyzing may be further based on the therapy program. Further, the notification may include a compliance notification. Further, the method 700 further may include transmitting, using the communication device, the compliance notification to at least one of the at least one patient device and the at least one external device.

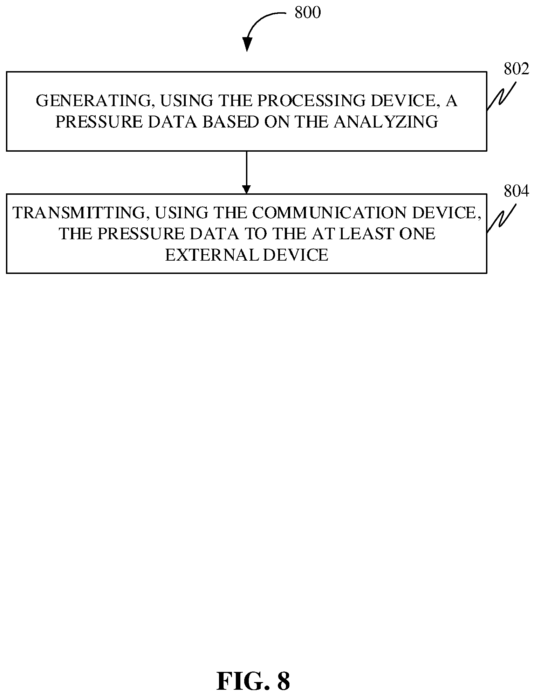

FIG. **8** is a flowchart of a method 800 for facilitating the generation of pressure data, in accordance with some embodiments. Further, at 802, the method 800 may include a step of generating, using the processing device, a pressure data based on the analyzing.

Further, at 804, the method 800 may include a step of transmitting, using the communication device, the pressure data to the at least one external device.

Further, in some embodiments, the sensor device may include a microprocessor, a wireless module, and a memory. Further, the microprocessor may be configured for performing at least one computational process. Further, the wireless module may be configured for connecting the sensor device to the at least one external device. Further, the memory may be configured for storing a data associated with the sensor device. Further, in some embodiments, the sensor device may include a non-intrusive device. Further, the non-intrusive device may be configured for allowing the flow upon failure of the sensor device.

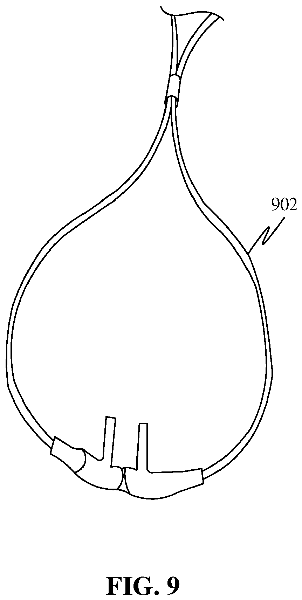

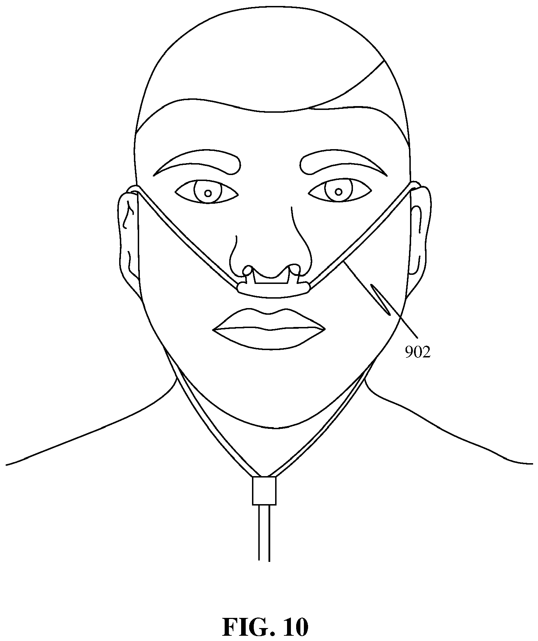

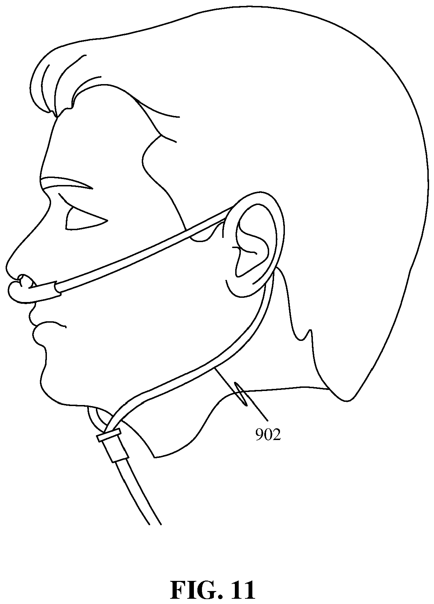

FIG. **9** is a top perspective view of a nasal cannula 902, in accordance with some embodiments. Further, the nasal cannula 902 is commonly used to deliver supplemental oxygen to patients in a hospital, home-care, nursing home, and other settings, who are in need of respiratory assistance. Further, the nasal cannula 902 may be attached to tubing which delivers oxygen from a wall connection in the hospital setting or from a portable oxygen generator in the home-care setting. Further, two prongs of the nasal cannula 902 may be placed in the patient's nostrils, as shown in FIG. **10** and FIG. **11****,** typically secured to the patient by wrapping the tubing around the patient's ears.

FIG. **10** is a front view of a user with the nasal cannula 902 in nostrils and tubing secured around ears, in accordance with some embodiments.

FIG. **11** is a side view of a user with the nasal cannula 902 in nostrils and tubing secured around ears, in accordance with some embodiments.

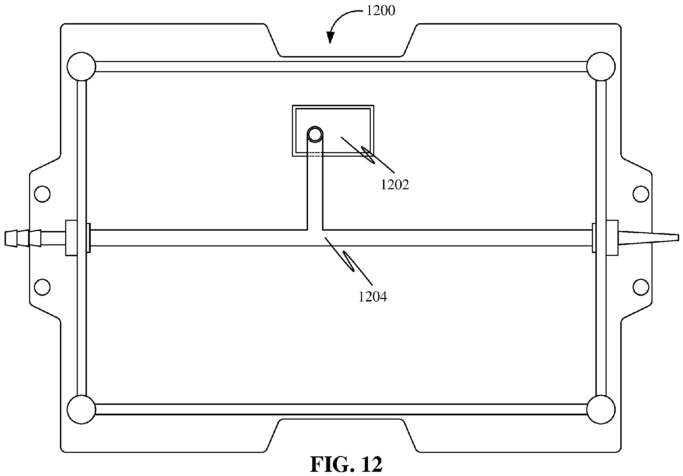

FIG. **12** is a top view of an oxygen delivery monitor (ODM) 1200, in accordance with some embodiments. Further, the oxygen delivery monitor (ODM) 1200 may monitor the delivery of supplemental oxygen through a nasal cannula to hospital and home-care patients. Further, the ODM 1200 may include both hardware and software components. Further, the hardware component may include an airflow sensor 1202 and a Bluetooth module (not shown) mounted on an open-source platform. Further, the software component consists of dedicated source-code apps that are compatible with both *iOS* and *Android* operating systems.

Further, the hardware component of the ODM 1200 may be introduced downstream of the oxygen supply and upstream of the nasal cannula. Further, the airflow sensor 1202 of the ODM 1200 may detect the pressure variation at the nasal cannula during a normal breathing cycle. Once a patient's baseline breathing pattern is established, the ODM 1200 may distinguish between normal and abnormal breathing. Further, the upstream end of the ODM 1200 may be connected to output tubing from the oxygen supply, and the downstream end may be connected to tubing leading to the nasal cannula. Oxygen may enter the upstream end of the ODM 1200 through an inflow valve. Further, the oxygen may flow through a "T" shaped tubing 1204, and pass over the airflow sensor 1202 to detect pressure variations without obstructing air flow, to an outflow valve on the downstream end of the ODM 1200. Further, the airflow sensor 1202 may continually measure oxygen flow-rate, which may be transmitted wirelessly via the Bluetooth module for evaluation of the patient's breathing pattern by the dedicated source code in a smartphone/computer app. Variation from normal breathing or a dislodged nasal cannula may manifest itself in variation of oxygen flow-rate through the airflow sensor 1202.

Further, the hardware component may be packaged in a clear plastic housing. Further, the hardware component may include a *Honeywell Zephry™HAF Series* airflow sensor and a *DF-Robot Bluno module* (*Arduino* with Bluetooth 4.0) mounted on a *DF-Robot* open-source hardware platform. Further, the ODM 1200 may be powered via a USB port (not shown) and may have an On/Off power switch.

Further, the ODM 1200 may continually monitor the patient's breathing pattern and alert the on-duty nurse, nurse's station or caregiver, via Bluetooth and smartphone/computer app, of an abnormality such as would result from a dislodged nasal cannula, unusually slow or fast breathing rates, pauses in breathing, or other abnormalities. Further, the ODM 1200 may also provide a record of such events.

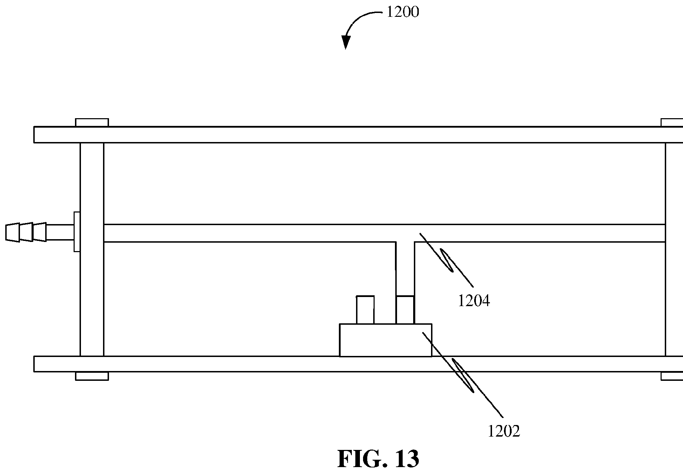

FIG. **13** is a front view of the ODM 1200, in accordance with some embodiments.

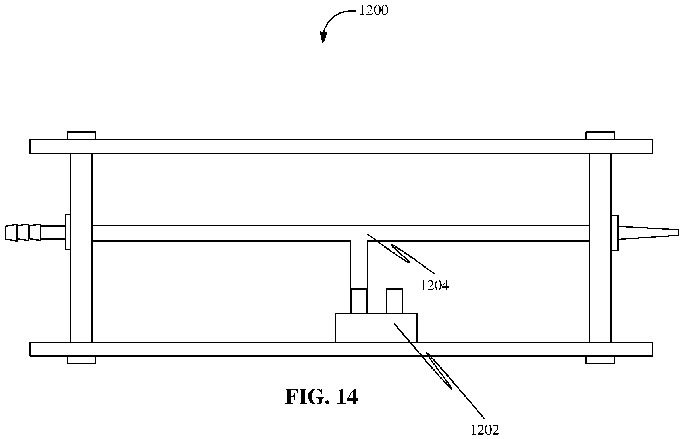

FIG. **14** is a rear view of the ODM 1200, in accordance with some embodiments.

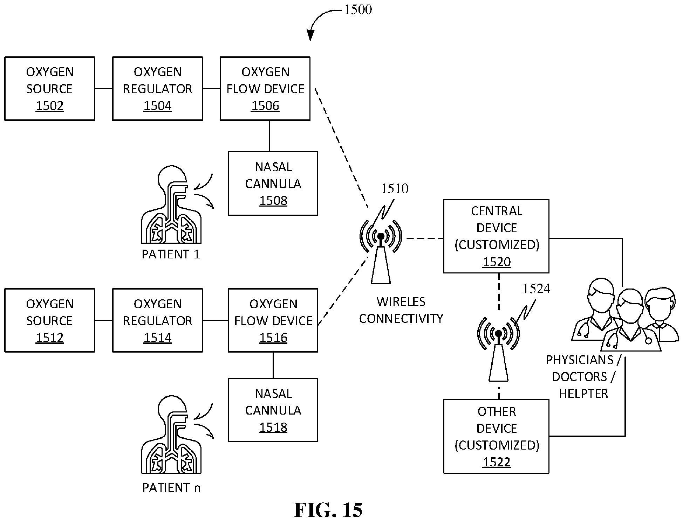

FIG. **15** is a block diagram of a system 1500 monitoring and management of a patient's oxygen therapy and breathing, in real time and for retrospective review, in accordance with some embodiments. Accordingly, the system 1500 may include an oxygen source 1502, an oxygen regulator 1504, and an oxygen flow device 1506 connected to a nasal cannula 1508 through a wireless connection 1510. Further, the system 1500 may include an oxygen source 1512, an oxygen regulator 1514, and an oxygen flow device 1516 connected to a nasal cannula 1518 through a wireless connection 1510. Further, the system 1500 may include a central device 1520 and other device 1522 connected through a wireless connection 1524.

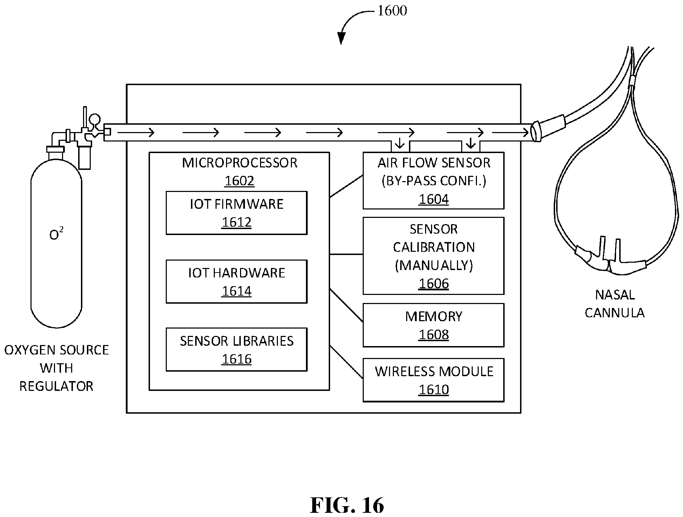

FIG. **16** is a block diagram of a system 1600 to facilitate monitoring and management of a patient's oxygen therapy and breathing, in real time and for retrospective review by an oxygen flow device, in accordance with some embodiments. Accordingly, the system 1600 may include a microprocessor 1602, an airflow sensor 1604, a sensor calibration 1606, a memory 1608, and a wireless module 1610. Further, the microprocessor 1602 may include an IoT firmware 1612, an IoT hardware 1614, and sensor libraries 1616.

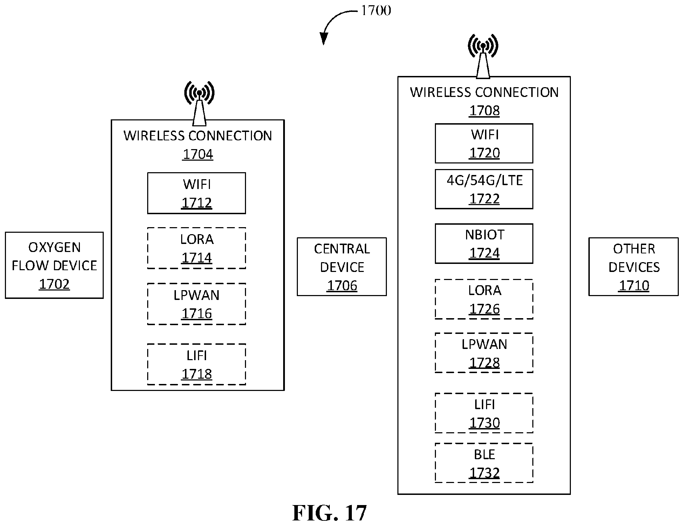

FIG. **17** is a block diagram of a system 1700 to facilitate data transmission associated with monitoring and management of a patient's oxygen therapy and breathing, in real time and for retrospective review, in accordance with some embodiments. Accordingly, the system 1700 may include an oxygen flow device 1702, a wireless connection 1704, a central device 1706, a wireless connection 1708, and other devices 1710. Further, the wireless connection 1704 may include Wi-Fi 1712, Lora 1714, LPWAN 1716, and LiFi 1718. Further, the wireless connection 1708 may include Wi-Fi 1720, 4G/5G/LTE 1722, NBIoT 1724, Lora 1726, LPWAN 1728, LiFi 1730, and BLE 1732.

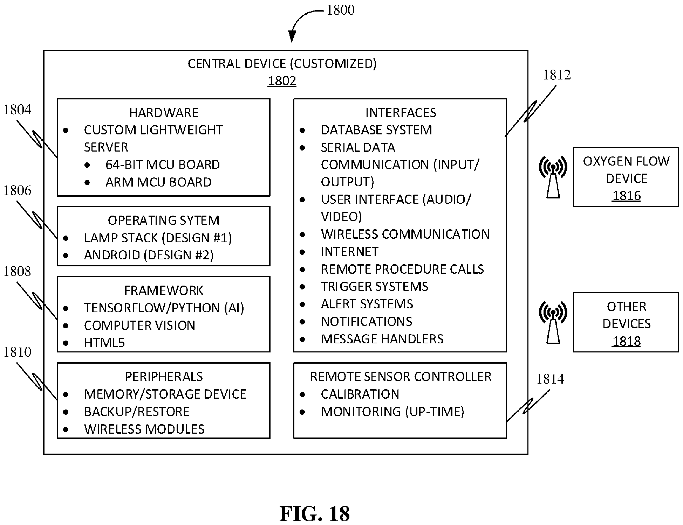

FIG. **18** is a block diagram of a system 1800 including a central device 1802 interacting with an oxygen flow device 1816 and other devices 1818 to facilitate monitoring and management of a patient's oxygen therapy and breathing, in real time and for retrospective review, in accordance with some embodiments. Accordingly, the system 1800 may include the central device 1802. Further, the central device 1802 may include a hardware 1804, an operating system 1806, a framework 1808, peripherals 1810, interfaces 1812, a remote sensor controller 1814.

Further, the hardware 1804 may include a custom lightweight server, a 64-bit MCU board, and an *ARM MCU* board. Further, the operating system 1806 may include a *LAMP* stack and *Android.Further,* the framework 1808 may include a *TensorFlow*/*python*(AI), computer vision, and *HTML5*.Further, the peripherals 1810 may include a memory/storage device, backup/restore, and wireless modules. Further, the interfaces 1812 may include a database system, a serial data communication (input/output), a user-interface (audio/video), a wireless communication, an internet, a remote procedure calls, a trigger system, an alert system, a notification, and a message handler. Further, the remote sensor controller 1814 may include calibration and monitoring (up-time). Further, the system 1800 may be connected to an oxygen flow device 1816 and other devices 1818 through a wireless connection.

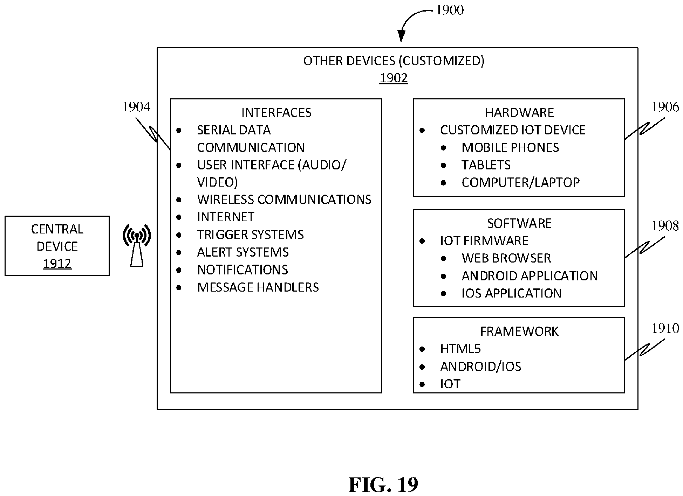

FIG. **19** is a block diagram of system 1900 including other devices 1902 interacting with a central device 1912 to facilitate monitoring and management of a patient's oxygen therapy and breathing, in real time and for retrospective review, in accordance with some embodiments. Accordingly, the system 1900 may include the other devices 1902. Further, the other devices 1902 may include interfaces 1904, hardware 1906, software 1908, and a framework 1910.

Further, the interfaces 1904 may include a serial data communication, a user interface (audio/video), a wireless communication, an internet, a trigger system, an alert system, a notification, a message handler. Further, the hardware 1906 may include a customized IoT device such as a mobile phone, a tablet, a computer/laptop. Further, the software 1908 may include an IoT firmware such as a web browser, an *Android* application, and an *iOS* application. Further, the framework 1910 may include *HTML5, AndroidliOS,* and IoT. Further, the system 1900 may be connected to a central device 1912 through a wireless connection.

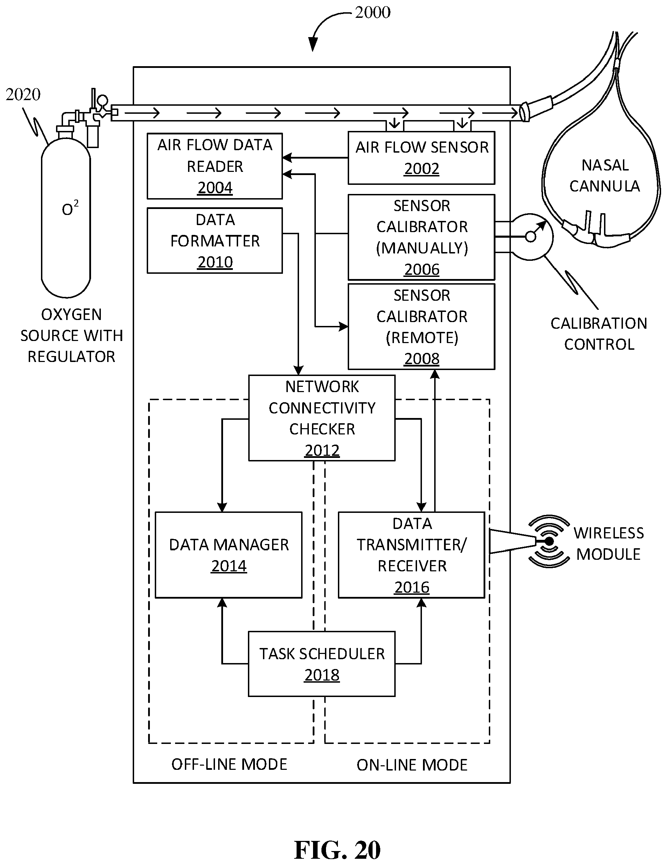

FIG. **20** is a block diagram of an oxygen flow device 2000 to facilitate monitoring and management of a patient's oxygen therapy and breathing, in real time and for retrospective review, in accordance with some embodiments. Accordingly, the oxygen flow device 2000 may include an airflow sensor 2002, an airflow data reader 2004, a sensor calibrator (manual) 2006, a sensor calibrator (remote) 2008, a data formatter 2010, a network connectivity checker 2012, a data manager 2014, a data transmitter/receiver 2016, and a task scheduler 2018.

Further, the airflow sensor 2002 may be always active. Further, the airflow data reader 2004 may convert airflow sensor readings into pressure data. Further, the sensor calibrator (manual) 2006 and the sensor calibrator (remote) 2008 may match the airflow sensor readings to oxygen source's regulator 2020. Further, the data formatter 2010 may convert airflow data into international standard units (For example - liters per minute [LPM]). Further, the network connectivity checker 2012 may monitor offline and online status of a device. Further, the data manager 2014 may control the sending/saving of data depending on network status. Further, the data transmitter/receiver 2016 may handle the in/out communication to a central device and other devices. Further, the task scheduler 2018 may manage internal/external events, trigger alerts, and send notifications.

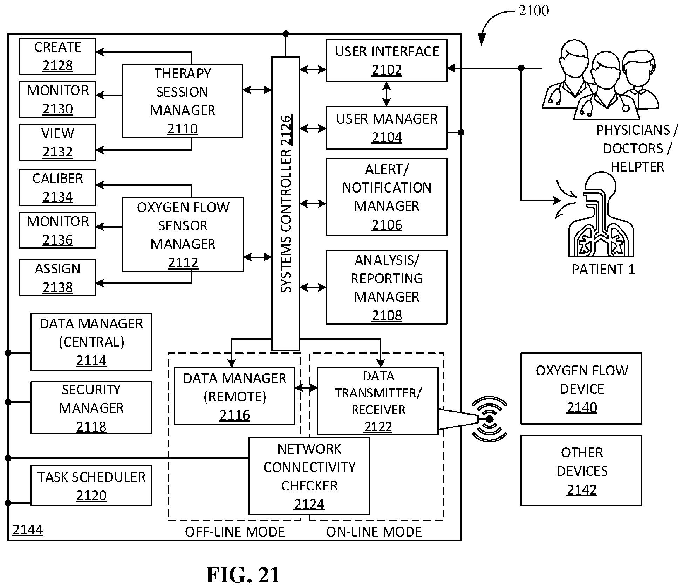

FIG. **21** is a block diagram of a system 2100 including a central device 2144 interacting with an oxygen flow device 2140 and other devices 2142 to facilitate monitoring and management of a patient's oxygen therapy and breathing, in real time and for retrospective review, in accordance with some embodiments. Accordingly, the system 2100 may include a user interface 2102, a user manager 2104, an alert/notification manager 2106, an analysis/reporting manager 2108, a therapy session manager 2110, an oxygen flow sensor manager 2112, a data manager (central) 2114, a data manager (remote) 2116, a security manager 2118, a task scheduler 2120, a data transmitter/receiver 2122, a network connectivity checker 2124, and systems controller 2126.

Further, the user interface 2102 may be a video/audio component that enables users to interact with the central device. Further, the user manager 2104 may be for creating/deleting users, access and privileges. Further, the alert/notification manager 2106 may be for creating/configuring triggers, events and action items. Further, the analysis/reporting manager 2108 may be for data retrieval, transformation, and aggregation. Further, the therapy session manager 2110 may be for creation 2128, monitor 2130, and view 2132 of records and on-going sessions. Further, the oxygen flow sensor manager 2112 may be for calibration 2134, monitor 2136, and assignment/designation 2138 processes. Further, the data manager (central) 2114 may be for creation, read, update, and archiving of the central device's data. Further, the data manager (remote) 2116 may be for creation, read, update, and archiving of a sensor device. Further, the security manager 2118 may be for system-wide protection against unauthorized access. Further, the task scheduler 2120 may be for the creation and execution of time-based processes. Further, the data transmitter/receiver 2122 may handle the in/out communication to sensors and other devices. Further, the network connectivity checker 2124 may monitor the offline and online status of the device. Further, the systems controller 2126 may be the overall systems manager/director. Further, the data transmitter/receiver 2122 may be connected to an oxygen flow device 2140 and other devices 2142 through a wireless connection.

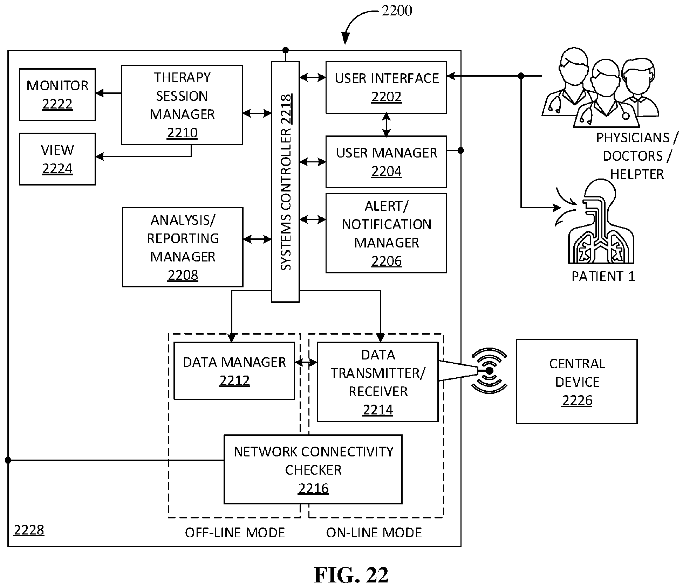

FIG. **22** is a block diagram of a system 2200 to facilitate monitoring and management of a patient's oxygen therapy and breathing, in real time and for retrospective review using other devices 2228, in accordance with some embodiments. Accordingly, the system 2200 may include a user interface 2202, a user manager 2204, an alert/notification manager 2206, an analysis/reporting manager 2208, a therapy session manager 2210, a data manager 2212, a data transmitter/receiver 2214, a network connectivity checker 2216, and systems controller 2218. Further, the data transmitter/receiver 2214 may be connected to a central device 2226 through a wireless connection.

Further, the therapy session manager 2210 may be for monitor 2222 and view 2224 of records and on-going sessions. Further, the functionality of the other device may be a trimmed-down version of the central device 2226. Further, the other device may be used for receiving alert and notification messages. Further, the other device may be used for browsing historical therapy sessions. Further, the other device may be used for analyzing therapy data and viewing reports. Further, the other device does not connect patient identity to the therapy session for security purposes and HIPAA compliance. Further, the other device does not allow data to be stored locally or forwarded to others for security purposes and HIPAA compliance.

Further, hardware options for the other devices 2228 may include a customized IoT device to secure the exclusivity of the solution. Further, the hardware options for the other devices 2228 may include off-the-shelf mobile devices for the reusability of existing devices that users may already have.

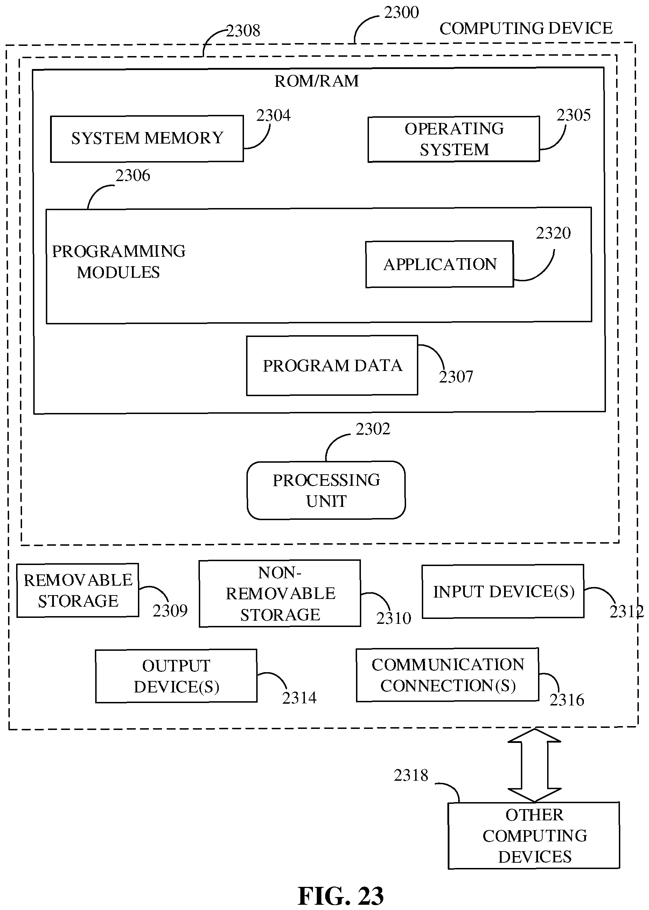

With reference to FIG. **23****,** a system consistent with an embodiment of the disclosure may include a computing device or cloud service, such as computing device 2300. In a basic configuration, computing device 2300 may include at least one processing unit 2302 and a system memory 2304. Depending on the configuration and type of computing device, system memory 2304 may comprise, but is not limited to, volatile (e.g. random-access memory (RAM)), non-volatile (e.g. read-only memory (ROM)), flash memory, or any combination. System memory 2304 may include operating system 2305, one or more programming modules 2306, and may include a program data 2307. Operating system 2305, for example, may be suitable for controlling computing device 2300's operation. In one embodiment, programming modules 2306 may include image-processing module, machine learning module. Furthermore, embodiments of the disclosure may be practiced in conjunction with a graphics library, other operating systems, or any other application program and is not limited to any particular application or system. This basic configuration is illustrated in FIG. **23** by those components within a dashed line 2308.

Computing device 2300 may have additional features or functionality. For example, the computing device 2300 may also include additional data storage devices (removable and/or non-removable) such as, for example, magnetic disks, optical disks, or tape. Such additional storage is illustrated in FIG. **23** by a removable storage 2309 and a non-removable storage 2310. Computer storage media may include volatile and non-volatile, removable and non-removable media implemented in any method or technology for storage of information, such as computer-readable instructions, data structures, program modules, or other data. System memory 2304, removable storage 2309, and non-removable storage 2310 are all computer storage media examples (i.e., memory storage.) Computer storage media may include, but is not limited to, RAM, ROM, electrically erasable read-only memory (EEPROM), flash memory or other memory technology, CD-ROM, digital versatile disks (DVD) or other optical storage, magnetic cassettes, magnetic tape, magnetic disk storage or other magnetic storage devices, or any other medium which can be used to store information and which can be accessed by computing device 2300. Any such computer storage media may be part of device 2300. Computing device 2300 may also have input device(s) 2312 such as a keyboard, a mouse, a pen, a sound input device, a touch input device, a location sensor, a camera, a biometric sensor, etc. Output device(s) 2314 such as a display, speakers, a printer, etc. may also be included. The aforementioned devices are examples and others may be used.

Computing device 2300 may also contain a communication connection 2316 that may allow device 2300 to communicate with other computing devices 2318, such as over a network in a distributed computing environment, for example, an intranet or the Internet. Communication connection 2316 is one example of communication media. Communication media may typically be embodied by computer-readable instructions, data structures, program modules, or other data in a modulated data signal, such as a carrier wave or other transport mechanism, and includes any information delivery media. The term "modulated data signal" may describe a signal that has one or more characteristics set or changed in such a manner as to encode information in the signal. By way of example, and not limitation, communication media may include wired media such as a wired network or direct-wired connection, and wireless media such as acoustic, radio frequency (RF), infrared, and other wireless media. The term computer-readable media as used herein may include both storage media and communication media.

As stated above, a number of program modules and data files may be stored in system memory 2304, including operating system 2305. While executing on processing unit 2302, programming modules 2306 (e.g., application 2320 such as a media player) may perform processes including, for example, one or more stages of methods, algorithms, systems, applications, servers, databases as described above. The aforementioned process is an example, and processing unit 2302 may perform other processes.

Generally, consistent with embodiments of the disclosure, program modules may include routines, programs, components, data structures, and other types of structures that may perform particular tasks or that may implement particular abstract data types. Moreover, embodiments of the disclosure may be practiced with other computer system configurations, including hand-held devices, general-purpose graphics processor-based systems, multiprocessor systems, microprocessor-based or programmable consumer electronics, application-specific integrated circuit-based electronics, minicomputers, mainframe computers, and the like. Embodiments of the disclosure may also be practiced in distributed computing environments where tasks are performed by remote processing devices that are linked through a communications network. In a distributed computing environment, program modules may be located in both local and remote memory storage devices.

Furthermore, embodiments of the disclosure may be practiced in an electrical circuit comprising discrete electronic elements, packaged or integrated electronic chips containing logic gates, a circuit utilizing a microprocessor, or on a single chip containing electronic elements or microprocessors. Embodiments of the disclosure may also be practiced using other technologies capable of performing logical operations such as, for example, AND, OR, and NOT, including but not limited to mechanical, optical, fluidic, and quantum technologies. In addition, embodiments of the disclosure may be practiced within a general-purpose computer or in any other circuits or systems.

Embodiments of the disclosure, for example, may be implemented as a computer process (method), a computing system, or as an article of manufacture, such as a computer program product or computer-readable media. The computer program product may be a computer storage media readable by a computer system and encoding a computer program of instructions for executing a computer process. The computer program product may also be a propagated signal on a carrier readable by a computing system and encoding a computer program of instructions for executing a computer process. Accordingly, the present disclosure may be embodied in hardware and/or in software (including firmware, resident software, micro-code, etc.). In other words, embodiments of the present disclosure may take the form of a computer program product on a computer-usable or computer-readable storage medium having computer-usable or computer-readable program code embodied in the medium for use by or in connection with an instruction execution system. A computer-usable or computer-readable medium may be any medium that can contain, store, communicate, propagate, or transport the program for use by or in connection with the instruction execution system, apparatus, or device.

The computer-usable or computer-readable medium may be, for example, but not limited to, an electronic, magnetic, optical, electromagnetic, infrared, or semiconductor system, apparatus, device, or propagation medium. More specific computer-readable medium examples (a non-exhaustive list), the computer-readable medium may include the following: an electrical connection having one or more wires, a portable computer diskette, a random-access memory (RAM), a read-only memory (ROM), an erasable programmable read-only memory (EPROM or Flash memory), an optical fiber, and a portable compact disc read-only memory (CD-ROM). Note that the computer-usable or computer-readable medium could even be paper or another suitable medium upon which the program is printed, as the program can be electronically captured, via, for instance, optical scanning of the paper or other medium, then compiled, interpreted, or otherwise processed in a suitable manner, if necessary, and then stored in a computer memory.

Embodiments of the present disclosure, for example, are described above with reference to block diagrams and/or operational illustrations of methods, systems, and computer program products according to embodiments of the disclosure. The functions/acts noted in the blocks may occur out of the order as shown in any flowchart. For example, two blocks shown in succession may, in fact, be executed substantially concurrently or the blocks may sometimes be executed in the reverse order, depending upon the functionality/acts involved.

While certain embodiments of the disclosure have been described, other embodiments may exist. Furthermore, although embodiments of the present disclosure have been described as being associated with data stored in memory and other storage mediums, data can also be stored on or read from other types of computer-readable media, such as secondary storage devices, like hard disks, solid-state storage (e.g., USB drive), or a CD-ROM, a carrier wave from the Internet, or other forms of RAM or ROM. Further, the disclosed methods' stages may be modified in any manner, including by reordering stages and/or inserting or deleting stages, without departing from the disclosure.

Although the present disclosure has been explained in relation to its preferred embodiment, it is to be understood that many other possible modifications and variations can be made without departing from the spirit and scope of the disclosure.

## Claims

1. A system for monitoring and management of a patient's oxygen therapy and breathing, wherein the system comprising:
a sensor device configured for detecting a flow, wherein the sensor device is configured to be fluidly couplable with a nasal cannula, wherein the nasal cannula is configured for dispensing oxygen to a nasal cavity of a patient, wherein the sensor device is configured for generating at least one sensor data based on detection of the flow;
a processing device communicatively coupled with the sensor device, wherein the processing device is configured for:
analyzing the at least one sensor data; and
generating a notification based on the analyzing;
a communication device communicatively coupled with the processing device, wherein the communication device is configured for transmitting the notification to at least one external device; and
a storage device communicatively coupled with the processing device, wherein the storage device is configured for storing the notification.

2. The system of claim 1 further comprising a presentation device communicatively coupled with the processing device, wherein the presentation device is configured for presenting the notification.

3. The system of claim 1, wherein the processing device is configured for:
determining a patient respiration data based on the analyzing;
analyzing the patient respiration data; and
generating an alert based on the analyzing of the patient respiration data, wherein the communication device is configured for transmitting the alert to the at least one external device.

4. The system of claim 3, wherein the storage device is configured for retrieving a patient data, wherein the processing device is configured for:
processing at least one of the patient data and the patient respiration data; and
generating a second notification based on the processing.

5. The system of claim 4, wherein the processing device is configured for generating a therapy program based on the processing, wherein the communication device is configured for transmitting the therapy program to at least one of at least one patient device and the at least one external device.

6. The system of claim 4, wherein the system is compliant with at least one Health Insurance Portability and Accountability Act (HIPPA) regulation, wherein the processing device is configured for deleting at least one of the at least one sensor data, the patient data, and the patient respiration data, wherein the deletion is subsequent to the analyzing.

7. The system of claim 5, wherein the analyzing is further based on the therapy program, wherein the notification comprises a compliance notification, wherein the communication device is configured for transmitting the compliance notification to at least one of the at least one patient device and the at least one external device.

8. The system of claim 1, wherein the processing device is configured for generating a pressure data based on the analyzing, wherein the communication device is configured for transmitting the pressure data to the at least one external device.

9. The system of claim 1, wherein the sensor device comprises a microprocessor, a wireless module, and a memory, wherein the microprocessor is configured for performing at least one computational process, wherein the wireless module is configured for connecting the sensor device to the at least one external device, wherein the memory is configured for storing a data associated with the sensor device.

10. The system of claim 9, wherein the sensor device comprises a non-intrusive device, wherein the non-intrusive device is configured for allowing the flow upon failure of the sensor device.

11. A method for monitoring and management of a patient's oxygen therapy and breathing, wherein the method comprising:
receiving, using a communication device, at least one sensor data from a sensor device, wherein the sensor device configured for detecting a flow, wherein the sensor device is configured to be fluidly couplable with a nasal cannula, wherein the nasal cannula is configured for dispensing oxygen to a nasal cavity of a patient;
analyzing, using a processing device, the at least one sensor data;
generating, using the processing device, a notification based on the analyzing;
transmitting, using the communication device, the notification to at least one external device; and
storing, using a storage device, the notification.

12. The method of claim 11 further comprising:
transmitting, using the communication device, the notification to a presentation device, wherein the presentation device is configured for presenting the notification.

13. The method of claim 11 further comprising:
determining, using the processing device, a patient respiration data
based on the analyzing;
analyzing, using the processing device, the patient respiration data;
generating, using the processing device, an alert based on the analyzing of the patient respiration data; and
transmitting, using the communication device, the alert to the at least one external device.

14. The method of claim 13 further comprising:
retrieving, using the storage device, a patient data;
processing, using the processing device, at least one of the patient data and the patient respiration data; and
generating, using the processing device, a second notification based on the processing.

15. The method of claim 14 further comprising:
generating, using the processing device, a therapy program based on the processing; and
transmitting, using the communication device, the therapy program to at least one of at least one patient device and the at least one external device.

16. The method of claim 14, wherein the method is compliant with at least one Health Insurance Portability and Accountability Act (HIPPA) regulation, the method further comprising deleting, using the processing device, at least one of the at least one sensor data, the patient data, and the patient respiration data, wherein the deleting is subsequent to the analyzing.

17. The method of claim 15, wherein the analyzing is further based on the therapy program, wherein the notification comprises a compliance notification, wherein the method further comprising transmitting, using the communication device, the compliance notification to at least one of the at least one patient device and the at least one external device.

18. The method of claim 11 further comprising:
generating, using the processing device, a pressure data based on the analyzing; and
transmitting, using the communication device, the pressure data to the at least one external device.

19. The method of claim 11, wherein the sensor device comprises a microprocessor, a wireless module, and a memory, wherein the microprocessor is configured for performing at least one computational process, wherein the wireless module is configured for connecting the sensor device to the at least one external device, wherein the memory is configured for storing a data associated with the sensor device.

20. The method of claim 19, wherein the sensor device comprises a non-intrusive device, wherein the non-intrusive device is configured for allowing the flow upon failure of the sensor device.
